# EUROPEAN PATENT APPLICATION

(11) **EP 2 468 236 A2**
(43) Date of publication of application: **27.06.2012**
(21) Application number: 11178523.4
(22) Date of filing: 23.08.2011
(51) Int. Cl.: A61K 8/04, A61K 8/19, A61K 8/81, A61Q 11/00

(54) **Aqueous oral care product**

(30) Priority: 23.12.2010 EP 10196729; 21.01.2011 EP 11151697; 05.05.2011 EP 11164961
(71) Applicant: Bio-Gate AG, 90411 Nürnberg (DE)
(72) Inventor: Steinrücke, Peter, 90461 Nürnberg (DE)
(74) Representative: Ehnis, Tobias

(57) **Abstract**

The invention refers to an aqueous oral care product comprising particles which consist of metal and which contain metallic silver, and a completely polymerized copolymer of maleic acid or derivative thereof and alkyl vinyl ether or N-vinylpyrrolidone or acrylic acid or acrylic acid ester.

## Description

The invention concerns an aqueous oral care product comprising a copolymer.

From US 5,334,375 an oral composition containing a substantially water insoluble noncationic antibacterial antiplaque agent and a synthetic cross-linked polymer such as cross-linked poly (methylvinyl ether/maleic acid or anhydride) as antibacterial-enhancing agent to enhance delivery of said antibacterial agent to, and the retention thereof on, oral surfaces is known. As noncationic antibacterial antiplaque agent an agent comprising a halogenated diphenyl ether, in particular triclosan, is disclosed. However, triclosan is known to be able to enrich in fat tissue of the human body and the use of triclosan is not undisputed among toxicologists.

Polymers as those disclosed in this patent application are distributed under the name "Gantrez" by International Specialty Products (ISP, Wayne, New Jersey, USA). All of these polymers are negatively charged at pH 7. They are used as adhesive resins for toothpaste. The negative charge supports an adhesion to the surface of teeth. If a cationic antibacterial agent or antibacterial agent comprising a cationic group would be used together with these polymers this agent could weaken the adhesion to the teeth.

From EP 1 668 052 B1 a hydrophilic, crosslinkable oligomer composition comprising a first oligomer and a second oligomer is known. The first oligomer comprises a plurality of polymerized monomer units having pendent, free-radically polymerizable functional groups, and a plurality of polymerized monomer units having pendent, hydrophilic poly (alkylene oxide) groups. The second oligomer comprises a plurality of polymerized monomer units having pendent photoinitiator groups. The first oligomer and second oligomer can be photochemically crosslinked by forming covalent bonds between said pendent, free-radically polymerizable functional groups of said first oligomer and said photoinitiator groups of said second oligomer. The oligomer composition may be used for wound dressings or as material for dental moldings or impressions. When the composition is used to prepare hydrophilic gel materials for medical applications, the gel can include one or more active agents such as antibacterial agents like antibiotics, iodine, silver, silver chloride and chlorhexidine. Other additives that can be incorporated into the gel material are viscosity modifiers such as polymeric thickeners, e.g. Gantrez resin. After crosslinking of the oligomer composition the Gantrez resin is fixed within the composition.

From EP 1 658 041 B1 a body care product containing porous particles formed of metal and containing metallic silver the mean diameter of which is between 1 and 100 µm is known. The body care product may be a toothpaste. The effect of the silver in such a toothpaste is restricted to the time of use of the toothpaste and a short time thereafter.

The object of the present invention is to provide an oral care product having a long lasting antibacterial effect such as the oral composition known from US 5,334,375 but without the disadvantages of the halogenated diphenyl ether such as triclosan.

The object is achieved by the features of claim 1. Embodiments of the invention are characterized by the features of claims 2 to 12.

According to the invention an aqueous oral care product comprising particles which consist of metal and which contain metallic silver, and a completely polymerized copolymer is provided. "Completely polymerized" means that polymerization has been completed and no further chain elongation takes place.

The copolymer is a copolymer of maleic acid or derivative thereof and alkyl vinyl ether or alkyl alcohol vinyl ether or N-vinylpyrrolidone or acrylic acid or acrylic acid ester comprising the repeating structural unit 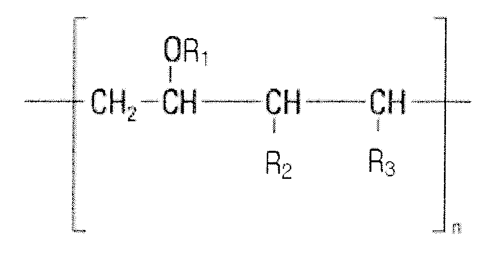 or 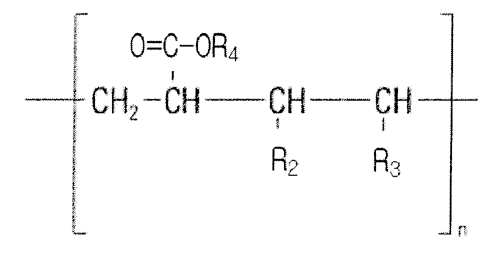 or 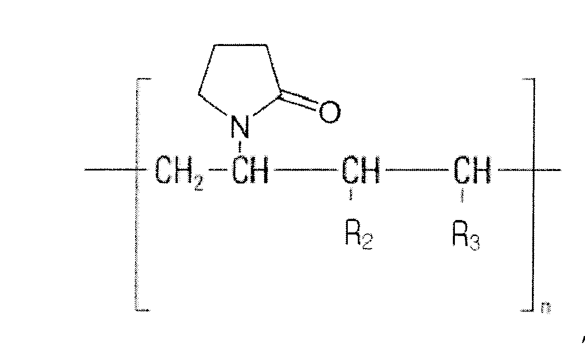 wherein R₁ represents a C₁ - C₄ alkyl group or C₁ - C₄ alkyl alcohol group,
wherein R₂ and R₃ represent independently from each other a COOR₅ - or a SO₂OR₆-group, wherein R₅ and R₆ represent independently from each other hydrogen, a C₁ - C₄ alkyl group or C₁ - C₄ alkyl alcohol group or another C₁ - C₄ aliphatic hydrocarbon,
and wherein R₄ represents hydrogen or a C₁ - C₄ alkyl group or C₁ - C₄ alkyl alcohol group or another C₁ - C₄ aliphatic hydrocarbon,
wherein n is an integer greater than 1 representing the number of repeat occurrences of this structural unit in a molecule of this copolymer and wherein the molecular weight of the copolymer is 80.000 to 2.000.000, wherein at least R₂ and/or R₃ are negatively charged at pH 7. Accordingly, R₂ or R₃ can be present as free acid that is dissociated at pH 7 or as an ester.

"Aqueous" means that the oral care product is contained in a composition comprising water in an amount exceeding trace amounts. The copolymer provides a film. Supported by the negative charge of R₂ and/or R₃ the film is secured against the dental surface.

The aqueous oral care product according to the invention does not have the disadvantages of an oral care product containing triclosan. Instead of bringing an antimicrobial in high concentration into contact with the oral cavity non-antimicrobial metallic particles containing silver are retained by the copolymer. Silver as such is not an antimicrobial. The particles are continuously producing silver ions, which are antimicrobially active and can also behave anti-inflammatorily. This makes it possible to create a depot using metallic silver, without the need to maintain a high local silver ion concentration that could impair the beneficial effect of the negatively charged copolymer.

Though the silver ions are cations that should bind to the negatively charged groups of the copolymer and thereby weaken adherence of the copolymer to the teeth inventors surprisingly found that the metallic particles are very effectively retained by the copolymer on the teeth and can provide an antimicrobial and anti-inflammatory effect.

The reason for that may be that the tendency of the silver ions to bind to the negatively charged groups of the copolymer is much lower than the high tendency to react with sulfhydryl groups of proteins in tissue, bacteria and saliva. As a consequence of this high reactivity with proteins and in contrast to triclosan silver ions do not effectively penetrate deep layers of oral mucosa and do not effectively enter the blood stream. As a further consequence the oral care product according to the invention may provide, with respect to the concentration required to achieve an antimicrobial effect, a relatively high local concentration of silver ions on the teeth and gingiva accompanied by a high antimicrobial and anti-inflammatory effect in this area. In contrast to this triclosan can penetrate skin and mucosa and enter into blood. It might therefore more severely affect the oral germ flora than silver ions. In addition triclosan is able to enriche in fat tissue and may be degraded in the organism into toxic products.

Though the antibacterial agent should be noncationic according to US 5,334,375 inventors found that an antibacterial substance producing cations can be used to provide an antibacterial effect without preventing an effective adhesion to teeth. Inventors found that locally produced ions prefer reacting with protein structures instead of binding to the copolymer. The copolymer comprising silver ions releasing particles is more effective in providing a long lasting antimicrobial effect to teeth than the same copolymer comprising only silver ions that neutralize the negative charge and thereby weaken binding of the copolymer to teeth.

In one embodiment of the invention R₁ is a methyl group. R₂ may be a carboxy group and/or R₃ may be a carboxy group, a COOC₂H₅ group or a COOC₄H₉ group.

According to a further embodiment the mean outer diameter of the single particles is 10 nm to 100 µm, in particular 1 to 50 µm, in particular 10 to 40 µm.

The particles may have a mean internal porosity of at least 65%, in particular between 65 and 95%, in particular between 65 and 90%, in particular between 70 and 85%, in particular between 75 and 85%, or between 85 and 95%, in particular between 90 and 95%.

Internal porosity is understood as meaning the percentage of the volume of the particle which is not filled with metal. The mean internal porosity of the particles can be determined using the following method:
1. Embedding the particles in a plastic,
2. preparing ultrathin sections of the embedded particles,
3. taking transmission electron microscopic (TEM) photographs of the particles,
4. determining the percentage of the area within each particle which is not filled with metal, in relation to the total area of this particle, in a plurality of TEM photographs, and
5. calculating the mean of a plurality of percentages which have been determined in this way.

In this connection, step 4 can be effected by means of a computer-assisted image analysis of the TEM photographs. In addition to the internal porosity, it is also possible to determine the total porosity of the particles. For this, the beat density of a powder of the particles is first of all determined. The beat density is the mass of a unit volume of a powder which is layered as densely as possible by means of beating. The beat density can be determined in accordance with DIN ISO 3953. The value which is determined in this connection is calculated as a percentage of the density of the metal forming the particles and subtracted from 100%. The value which is calculated in this way constitutes the total porosity of the particles.

The choice of the porosity can be used to specify the quantity of silver ions which are released by a particle in a particular unit of time. If a high porosity is chosen, this thereby releases many silver ions which means that, overall, the antimicrobial and antiinflammatory effect is achieved using a lower quantity of silver in the oral care product.

The particles may consist of metallic silver to an extent of at least 99% (w/w), in particular of at least 99.9% (w/w).

The oral care product according to the invention may further comprise at least one additive selected from the group consisting of sodium carboxymethylcellulose, waxes, oils, preservatives, flavoring agents, viscosity modifiers, sweeteners, colorants, polyphosporic acids, quaternary ammonium compounds, biguanides, surfactants, whitening agents, anticaries agents, tartar control agents, antiplaque agents, periodontal actives, abrasives, breath freshening agents, malodor control agents, tooth desensitizers, salivary stimulants orally acceptable carriers, and mixtures thereof.

The oral care product according to the invention may be formulated as a paste, gel, or liquid. The oral care product may be a mouthrinse or a dentifrice, in particular a toothpaste or a toothgel.

The invention further relates to the oral care product according to the invention for use as a medicament. In particular the oral care product according to the invention may be for the treatment of ailment caused by germs in the oral cavity. The ailment may be periodontosis, gingivitis, dental plaque, stomatitis, or halitosis.

### Embodiments:

### Example 1 :

An aqueous oral care product according to the invention has been mixed from the following components in the following proportions:
Abrasive silica 12% (w/w),
thickening silica 8% (w/w),
70% (w/w) sorbitol 35% (w/w),
cellulose gum 0.4% (w/w),
86% (w/w) glycerine 15% (w/w),
Gantrez S 2% (w/w),
sodium lauroyl sarcosinate 1.5% (w/w),
sodium saccharinate 0.1% (w/w),
titanium dioxide 0.8% (w/w),
aroma 0.8% (w/w),
sodium fluoride 0.25% (w/w),
potassium sorbate 0.2% (w/w),
porous silver particles (average diameter about 10 µm)
0.05% (w/w) and
water ad 100% (w/w);
pH adjusted to 4.9;

### Example 2:

A further aqueous oral care product according to the invention has been mixed from the following components in the following proportions:
70% (w/w) sorbitol 24% (w/w),
hydrated silica 18% (w/w),
85% (w/w) glycerine 5% (w/w),
pyrogenic silica 2% (w/w),
titanium dioxide 2% (w/w),
Gantrez S 2% (w/w),
sodium lauroyl sarcosinate 1.5 % (w/w),
cellulose gum 1 % (w/w),
zinc gluconate 1% (w/w),
aroma 1% (w/w),
potassium sorbate 0.3% (w/w),
sodium fluoride 0.25% (w/w),
sodium saccharinate 0.2% (w/w),
porous silver particles (average diameter about 10 µm) 0.05% (w/w) and
water ad 100% (w/w);
pH adjusted to 4.9

"Gantrez S" is the commercial name of a copolymer of maleic acid and methyl vinyl ether distributed by International Specialty Products (ISP, Wayne, New Jersey, USA). The porous silver particles are distributed by Bio-Gate AG, Neumeyerstr. 28 - 34, 90411 Nuremberg, Germany under the commercial name "MicroSilver BG".

The oral care product according to example 2 has been used in a clinical study with two groups of test persons. Group 1 used the oral care product according to example 2 for oral care. Group 2 used the same composition but without Gantrez S. Two and 14 hours after the application of the oral care product samples of the dental plaque were taken from the test persons. Using inductively coupled plasma mass spectrometry (ICP-MS) the samples were examined for their content of silver to evaluate which percentage of the silver contained in the sample of dental plaque taken after two hours is still present in the sample of the dental plaque taken after 14 hours of the same test person.

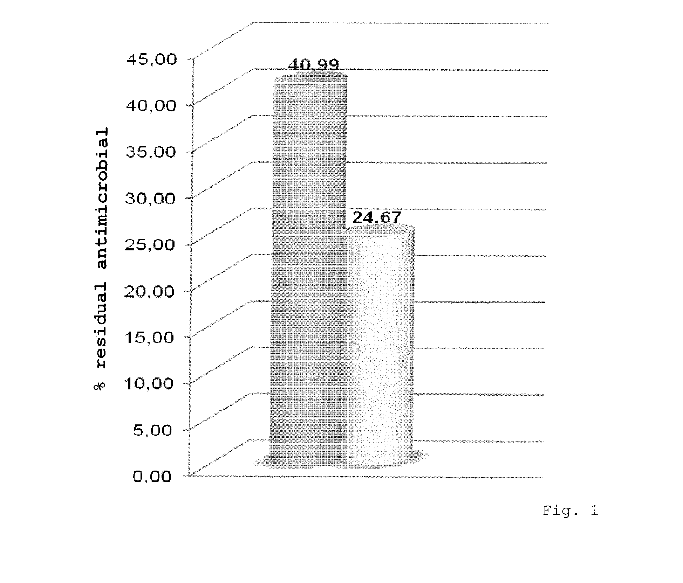

Fig. 1 shows the result of this study. The right column of Fig. 1 represents the average percentage of silver in the samples of dental plaque taken from test persons of group 2 and the left column the average percentage of silver in the samples of dental plaque taken from test persons of group 1. As can be seen from this result the presence of Gantrez S increased the percentage of silver retained on the teeth.

## Claims

1. Aqueous oral care product comprising particles which consist of metal and which contain metallic silver, and a completely polymerized copolymer of maleic acid or derivative thereof and alkyl vinyl ether or alkyl alcohol vinyl ether or N-vinylpyrrolidone or acrylic acid or acrylic acid ester comprising the repeating structural unit 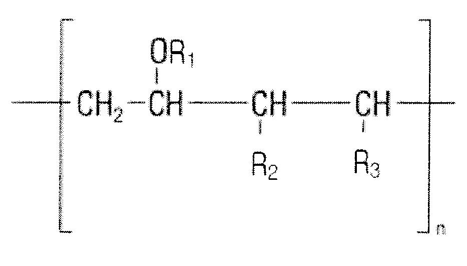 or 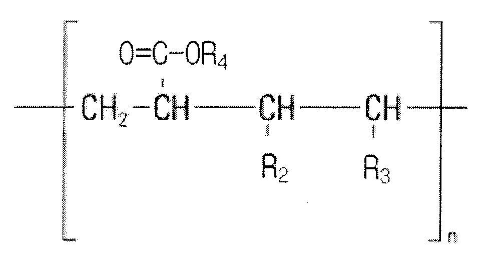 or 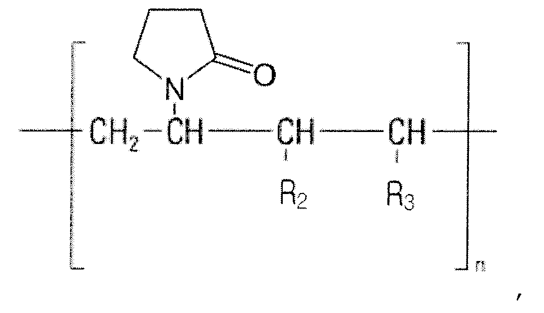 wherein R₁ represents a C₁ - C₄ alkyl group or C₁ - C₄ alkyl alcohol group,
wherein R₂ and R₃ represent independently from each other a COOR₅ - or a SO₂OR₆-group, wherein R₅ and R₆ represent independently from each other hydrogen, a C₁ - C₄ alkyl group or C₁ - C₄ alkyl alcohol group or another C₁ - C₄ aliphatic hydrocarbon,
and wherein R₄ represents hydrogen or a C₁ - C₄ alkyl group or C₁ - C₄ alkyl alcohol group or another C₁ - C₄ aliphatic hydrocarbon,
wherein n is an integer greater than 1 representing the number of repeat occurrences of this structural unit in a molecule of this copolymer and wherein the molecular weight of the copolymer is 80.000 to 2.000.000, wherein at least R₂ and/or R₃ are negatively charged at pH 7.

2. The oral care product of claim 1, wherein R₁ is a methyl group.

3. The oral care product of one of the preceding claims, wherein R₂ is a COOH group and/or R₃ is a COOH group, a COOC₂H₅ group or a COOC₄H₉ group.

4. The oral care product of one of the preceding claims, wherein the mean outer diameter of the single particles is 10 nm to 100 µm, in particular 1 to 50 µm, in particular 10 to 40 µm.

5. The oral care product of one of the preceding claims, wherein the particles have a mean internal porosity of at least 65%, in particular between 65 and 95%, in particular between 65 and 90%, in particular between 70 and 85%, in particular between 75 and 85%, or between 85 and 95%, in particular between 90 and 95%.

6. The oral care product of one of the preceding claims, wherein the particles consist of metallic silver to an extent of at least 99% (w/w), in particular of at least 99.9% (w/w).

7. The oral care product of one of the preceding claims further comprising at least one additive selected from the group consisting of sodium carboxymethylcellulose, waxes, oils, preservatives, flavoring agents, viscosity modifiers, sweeteners, colorants, polyphosporic acids, quaternary ammonium compounds, biguanides, surfactants, whitening agents, anticaries agents, tartar control agents, antiplaque agents, periodontal actives, abrasives, breath freshening agents, malodor control agents, tooth desensitizers, salivary stimulants orally acceptable carriers, and mixtures thereof.

8. The oral care product of one of the preceding claims formulated as a paste, gel, or liquid.

9. The oral care product of one of the preceding claims formulated as a mouthrinse or a dentifrice, in particular a toothpaste or a toothgel.

10. The oral care product of one of the preceding claims for use as a medicament.

11. The oral care product of one of claims 1 to 9 for the treatment of an ailment caused by germs in the oral cavity.

12. The oral care product of claim 11, wherein the ailment is periodontosis, gingivitis, dental plaque, stomatitis, or halitosis.
